# EUROPÄISCHE PATENTANMELDUNG

(11) **EP 1 321 122 A1**
(43) Veröffentlichungstag der Anmeldung: **25.06.2003**
(21) Anmeldenummer: 01130634.7
(22) Anmeldetag: 21.12.2001
(51) Int. Cl.: A61K 7/06, A61K 7/48, A61K 7/00, A61K 35/00

(54) **Heil- und Pflegemittel auf Basis von Sauerteig sowie Verfahren zu seiner Herstellung**

(71) Anmelder: woresan GmbH, 30916 Isernhagen (DE)
(72) Erfinder: Die Erfindernennung liegt noch nicht vor
(74) Vertreter: Meyers, Hans-Wilhelm, Dr.Dipl.-Chem.

(57) **Zusammenfassung**

Verfahren zur Herstellung eines Heil- und Pflegemittels für die Haut auf Basis eines ausgesäuerten Sauerteiges, wobei
1.1 einem Roggenmehl oder Roggenschrot ein natürlicher enzymatischer Starter wie α-Amylase und Wasser zugegeben wird, die Mischung einem Erhitzungsprozess auf 65 -75°C innerhalb von 3-5 Stunden ausgesetzt wird, wobei durch enzymatische Reaktionen eine starke Maltosebildung einsetzt,
1.2. eine weitere Zugabe von Roggenmehl oder Roggenschrot, Wasser sowie eines bakteriologischen Impfguts aus der Gruppe der heterofermentativen Milchsäurebakterien, vorzugsweise Lactobacillus DSM 6037 und Lactobacillus DSM 6129, erfolgt
1.3. und der Ansatz bis zur Einstellung der Stoffwechseltätigkeit der Mikroorganismen ausgesäuert wird.

## Beschreibung

Die Erfindung betrifft ein Verfahren zur Herstellung eines Heil- und Pflegemittels für die Haut auf Basis eines ausgesäuerten Sauerteiges, ein Heil- und Pflegemittel erhältlich nach diesem Verfahren, ein Kosmetikum enthaltend ein Heil- und Pflegemittel sowie die Verwendung des erfindungsgemäßen Heil- und Pflegemittels in einem Kosmetikum.

Aus der DE 38 02 840 02 ist bereits. ein Verfahren zur Herstellung eines Milchsäure und lebensfähige Milchsäurebakterien enthaltenden Gärproduktes durch Versäuern einer wässrigen Aufschlämmung von gebackener Brotmasse auf Sauerteigbasis in milchsaurem Medium bekannt geworden, wobei als Brotmasse das Backprodukt eines ausgesäuerten Brotteiges eingesetzt wird. Dieses Gärprodukt soll als u. a. als Hilfsmittel bei der Behandlung des ganzheitlichen Organismus von Mensch, Tier und Pflanze einsetzbar sein.

Aus der DE 38 46 186 A1 ist es auch bereits bekannt geworden, aus dem Gärschlamm der Spontanversäuerung von Sauerteigbroten gewonnene Präparate z. B. als Packungszuschlagstoff zur Behandlung von chronisch entzündlichen Krankheiten der Haut einzusetzen.

Durch die DT 26 11 972 B1 ist es bereits bekannt geworden, zum Herstellen eines Sauerteigs für die Bereitung von Broten und Backwaren aus Getreideschroten und/oder Mehlen in der den Vorteig bildenden Getreidemaische die Sauerteigbakterien bis zur Einstellung jeglicher bakteriologischer Stoffwechselaktivität auszusäuern.

In der EP 0 530 861 B1 ist eine topische, antimikrobielle pharmazeutische Zusammensetzung offenbart, die ein Gemisch aus C₆- bis C₁₈-Fettsäuren umfasst.

DE 299 23 627 U1 betrifft ein Heil- und Pflegemittel zur Förderung der Durchblutung, zur Lösung von Muskelverspannungen, zur Hautreinigung und zur Hautregenerierung, das auf Körperteile aufgetragen oder als Zusatz zu einem Wasserbad verwendet wird. Das Produkt ist dadurch gekennzeichnet, dass es von einem Fermentationsprodukt eines ausgesäuerten Sauerteiges gebildet ist.

Natürliche durch Fermentation gebildete Produkte, die mit der Haut in Kontakt gebracht werden besitzt ein Potential, pflegebedürftige Haut positiv zu beeinflussen.

Aufgabe der vorliegenden Erfindung war es, die positiven Einflüsse, die insbesondere aus der DE 299 23 627 U1 bekannten Wirkungen verbessern. Überraschenderweise sind Fermentationsprodukte von Roggenschrot oder Roggenmehlen mit heterofermentativen Milchsäurebakterien, die vor der Fermentation mit α-Amylase behandelt worden sind, geeignet, die gestellte Aufgabe zu lösen.

Gegenstand der vorliegenden Erfindung ist ein Verfahren zur Herstellung eines Heil- und Pflegemittels für die Haut aus Fermentationsprodukten auf Basis eines ausgesäuerten Sauerteiges, wobei
1.1 einem Roggenmehl oder Roggenschrot ein natürlicher enzymatischer Starter wie α-Amylase und Wasser zugegeben wird, die Mischung einem Erhitzungsprozess auf 65 -75°C innerhalb von 3-5 Stunden ausgesetzt wird, wobei durch enzymatische Reaktionen eine starke Maltosebildung einsetzt,
1.2. eine weitere Zugabe von Roggenmehl oder Roggenschrot, Wasser sowie eines bakteriologischen Impfguts aus der Gruppe der heterofermentativen Milchsäurebakterien, vorzugsweise Lactobacillus DSM 6037 und Lactobacillus DSM 6129, erfolgt
1.3. und der Ansatz bis zur Einstellung der Stoffwechseltätigkeit der Mikroorganismen ausgesäuert wird.

In einer Weiterbildung des erfindungsgemäßen Verfahrens wird dem Fermentationsprodukt ein zweites kohlehydrathaltiges Substrat und Wasser sowie ein bakteriologisches Impfgut aus der Gruppe der heterofermentativen Milchsäurebakterien zugegeben und die Mischung ausgesäuert wird,
- der letzte Verfahrensschritt ggf. ein oder mehrmals wiederholt wird, wobei die Mischung abschließend bis zur Einstellung jeglicher bakteriologischer Stoffwechselaktivität ausgesäuert wird und
- das Fermentationsprodukt einem stabilisierenden Erhitzungsprozess nahe der Siedetemperatur unterzogen wird.

Gegenstand der Erfindung ist ein Heil- und Pflegemittel für die Haut auf Basis eines ausgesäuerten Sauerteiges erhältlich nach dem erfindungsgemäßen Verfahren.

Das erfindungsgemäße Heil- und Pflegemittel weist vorteilhafterweise einen hohen Anteil auf fermentativem Wege gewonnener Milchsäure von > 60 Gew.-% (als Anteil der Gesamtsäuremenge) auf.

Die erfindungsgemäßen Heil- und Pflegemittel sind mit in der Kosmetik üblichen Hilfs- und Trägerstoffen versetzbar, um Kosmetika herzustellen.

Die einsetzbaren erfindungsgemäßen Heil- und Pflegemittel werden insbesondere in Form von Pulvern, Roggenfluiden, Roggenseren, Roggengelen verwendet.

Das aus den erfindungsgemäßen Heil- und Pflegemittel herstellbare erfindungsgemäße Kosmetikum kann insbesondere in Form von Salben, Pulvern, Cremes, Lotionen, Gelen, Shampoos, Haarspülungen, Packungen, Lippenstiften, Gesichts- und Körpermasken, oder Kombinationen davon eingesetzt werden.

So kann beispielsweise eine Gesichtsmaske zur Reinigung hergestellt werden aus in kosmetischen Rezepturen geläufigen Inkredenzien, wie Steareth-12 oder Steareth-21, Glycerylstearaten, Stearinsäure, Alkylbenzoaten mit Kettenlängen von insbesondere 12 bis 15 Kohlenstoffatomen, Cetearylisononanoat, Stearylheptanuat, Octylstearat, Duft- oder Aromastoffen, wie beispielsweise Persea Gratissima Avocadol (Firma CosmoCare), dem erfindungsgemäßen Roggengel oder Roggenfluid, Propyleneglycol, Füllstoffen wie Kaolin und Wasser. Eine andere Anwendungsform kann bestehen aus überwiegend erfindungsgemäßem Serum mit hohem Milchsäuregehalt sowie Vitaminen und anderen Ergänzungsstoffen. Ein Shampoo kann typischerweise Inkredenzien enthalten, wie Sodiumlaurethsulfat, Dinatriumlaurethsulfosuccinat, Cocoamidopropylbetain, PEG-7 Glycerylcocoate, dem erfindungsgemäßen Roggenserum, Füllstoffen wie Styrolacrylatcopolymeren/Laureth-23, Zitronensäure, Polyquaternium-10 (Firma Amerchol), PEG-18 Glyceryloleat oder -cocoate sowie Wasser, Konservierungsstoffen und Duftstoffen. Eine typische Kurpackung kann bestehen aus Cetearylalkohol, Ceteareth-12, Ceteareth-20, PEG-75 Lanolin, Triticum Vulgare (Firma Dragoco), dem erfindungsgemäßen Roggenfluid oder Roggenserum, Hydroxycetyl oder Hedroxyethyl Dimonium Chlorith der Firma Henkel, Panthenol, Zitronensäure sowie Wasser, Konservierungsstoffe und Duftstoffe.

Ein Haarkonditionierer kann insbesondere bestehen aus dem erfindungsgemäßen Roggenfluid oder Roggenserum mit Duft-, Konservierungs- und Füllstoffen, beispielsweise Obstessig, Glycerin, Cetearylalkohol, Ceteareth-12, Cetrimoniumchlorith, PEG-75 Lanolin, Konservierungsstoffe, Duftstoffe sowie Wasser. Die genannten Inkredenzien sind in der Kosmetik üblicherweise eingesetzte Chemikalien und sämtlich kommerziell erhältlich.

In einer Ausführungsform der vorliegenden Erfindung wird das erfindungsgemäß Heil- und Pflegemittel in Form eines Roggenfluids eingesetzt. Dabei handelt es sich um einen cremigen aus Roggengel gewonnenen Rohstoff, der sich auszeichnet in Cremes, Lotionen, Shampoos, Haarspülungen und Packungen. Das erfindungsgemäße Roggengel ist ein eher dickflüssiger Rohstoff, der auch direkt als erfindungsgemäßes Heil- und Pflegemittel eingesetzt werden kann. Es handelt sich dabei um das mehr oder weniger unmittelbar aus dem Fermentationsprozess gewonnenen Produkt, was sich in unveränderter Form als natürliche Gesichts- oder Körpermaske sowie als Badezusatz einsetzen lässt. Vorteil an dieser Ausführungsform ist das Fehlen von den Organismus belastender möglicherweise unverträglicher Bestandteile. Nach Anwendung zeigen sich Verbesserungen des Hautreliefs und Verlangsamung der Bildung von Hautunreinheiten. Das Mittel kann als biologisches Peelingmittel abgestorbener Hautschuppen dienen, wobei Vorteilhafterweise ein Moisturizing-Effekt in Erscheinung tritt.

Aus Roggenfluid kann seinerseits ein Roggenserum gewonnen werden. Es eignet sich als Rohstoff zum Einarbeiten in Gesichts- und Körpermasken, Lippenstiften, Shampoos und Spülungen sowie Haarpackungen.

Ausgehend vom Roggengel können beispielsweise Roggenpulver, Roggenfluid sowie das Roggenserum hergestellt werden. Ausgehend von Roggengel kann das Roggenpulver hergestellt werden durch Separieren der Feststoffe aus dem erfindungsgemäßen Roggengel, das anschließend getrocknet und vermahlen wird. Die Feststoffe des Roggenpulvers können als Peelingkörper in den erfindungsgemäßen Kosmetika eingesetzt werden. Das Roggenfluid wird beispielsweise hergestellt durch Abtrennung vom gereinigten Gel und Anreicherung mit unfiltriertem Roggenserum bis die für die Verwendung geeignete Viskosität eingestellt ist. Es handelt sich bei diesem Produkt um einen Rohstoff mit hohem Wirkstoffanteil. Das Roggenserum wird beispielsweise hergestellt durch Filtrieren des flüssigen Überstandes des aus der Fermentation gewonnenen erfindungsgemäßen Roggengels und anschließende Klärung. Dabei entsteht ein Produkt mit sehr hohem Wirkstoffanteil.

Gegenstand der Erfindung ist auch die Verwendung der erfindungsgemäßen Heil- und Pflegemittel in einem Kosmetikum gemäß der Erfindung.

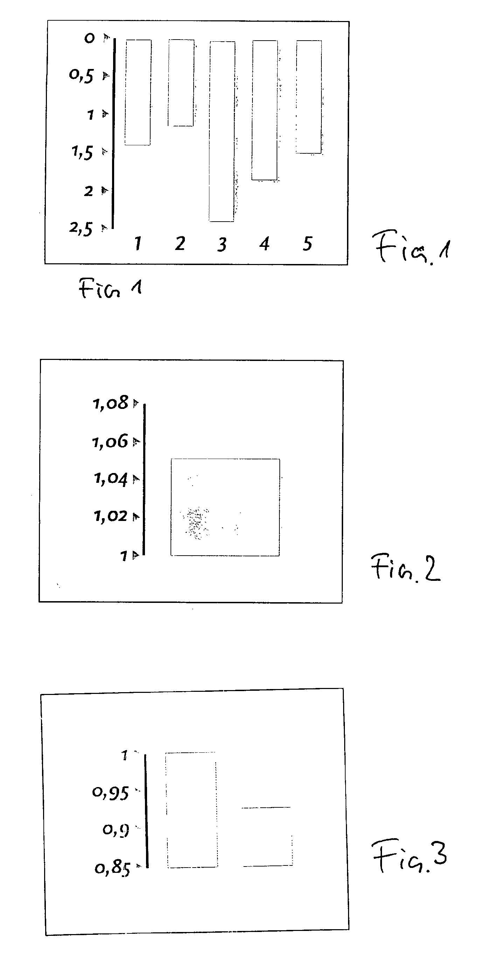

Fig. 1 zeigt die Beeinflussung des Erscheinungsbildes unreiner Haut durch die Behandlung mit einer Roggengelmaske. Die Reduzierung der Läsionen bei den Probanden 1 - 5 erfolgte um den Faktor bis zu 2,5.

Fig. 2 verdeutlicht den Moisturizing-Effekt. Es wird die Erhöhung der Hautfeuchtigkeit dargestellt.

Fig. 3 betrifft die Hautmikrotopographie und verdeutlicht die Reduktion der Haurauhigkeit bei zwei Probanden.

### Beispiel 1

Zur Herstellung des erfindungsgemäßen Roggengels wird ein Teil Roggenmehl 1150 mit zwei Teilen Wasser vermischt und einer Starterkultur mit α-Amylase versetzt. Die Mischung wird für 3 bis 5 Stunden auf 65 bis 75 °C erhitzt. Nach dem Start der enzymatischen Reaktion wird die Startfermentation eingeleitet durch Mischen von einem Teil Roggenmehl 1150 mit zwei Teilen Wasser. Dieser Mischung werden Fermentationsmedien mit Lactobacillus DSM 6037 sowie Lactobacillus DSM 6129 zugegeben. Diese Mischung wird für 24 bis 48 Stunden bei Temperaturen von 30 bis 34 °C fermentiert. Die Fermentation ist beendet, wenn sich ein konstanter pH-Wert einstellt. Die aus der Startfermentation gewonnene Mischung wird mit 87,5 Teilen, bezogen auf das Gesamtvolumen der Startfermentation, mit Wasser aufgefüllt. Die Fermentationszeit beträgt 24 bis 48 Stunden bis ein konstanter pH-Wert erreicht wird bei einer typischen Temperatur von 30 bis 34 °C. Danach erfolgt eine thermische Behandlung für 3 bis 5 Stunden bei einer Temperatur von 92 bis 100 °C. Gegebenenfalls werden Konservierungsmittel, wie Sorbinsäure und Benzoesäure, hinzugegeben. Typischerweise können 0,75 Teile Sorbinsäure bezogen auf das Gesamtvolumen eingesetzt werden. An den Konservierungsschritt schließen sich die Trennverfahren zur Herstellung der einzelnen Fraktionen, wie Roggenserum, Roggenfluid oder Roggenpulver an.

### Beispiel 2

Im folgenden wird im einzelnen auf einige Untersuchungen eingegangen.

Veränderungen der Anzahl der Auffälligkeiten in Relation zur unbehandelten Situation und zur Ausgangssituation.

### Anzahl der Komedonen und entzündlichen Läsionen

Die Behandlung mit dem Prüfmuster führte bei allen Probanden zu allen Meßzeitpunkten zur Reduktion der relativen Anzahl der entzündlichen Läsionen und Komedonen (Ausnahme Proband 1, Meßzeitpunkt 2 Wochen). Dabei sind Arealunterschiede und Veränderungen, die auf dem unbehandelten Areal z.B. durch klimatische Veränderungen oder individuelle Einflüssse zu verzeichnen waren, berücksichtigt. Unter Betrachtung aller Meßzeitpunkte unabhängig voneinander (Notwendigkeit zur Durchführung einer statistischen Betrachtung in Anbetracht der geringen Fallzahl) ergibt sich eine statistisch signifikante Reduktion (p < 0,05) sowohl der Anzahl der entzündlichen Läsionen als auch der Anzahl der Komedonen. Der Grad der Verbesserung der entzündlichen Läsionen und Komedonen ist relativ hoch. Eine Tendenz hinsichtlich der Dauer der Anwendung ist nicht erkennbar.

### Visuelle Beurteilung des Erscheinungsbildes der Haut

Das visuelle Erscheinungsbild der behandelten Gesichtshälfte stellte sich zu allen Meßzeitpunkten und bei allen Probanden leicht verbessert gegenüber der unbehandelten Gesichtshälfte dar. Der optische Unterschied betraf vorwiegend den Grad der Rötung und den Gesamteindruck sowie die Größe der einzelnen Auffälligkeiten.

### Subjektive Beurteilung durch den Probanden

Alle Probanden beurteilten die Wirkung des Prüfmusters als leicht bis eindeutig positiv. In 2 Fällen beschrieben die Probanden eine Verringerung der Hautrötung. 4 Probanden empfanden die behandelte Haut glatter als die unbehandelte. Eine Probandin beschrieb das Produkt als hautberuhigend.

Während der Anwendungphase empfanden 3 Probanden unmittelbar nach Produktauftrag eine negative sensuelle Empfindung in Form eines Spannens, leichten Prickeins bzw. Juckens.

### Diskussion

Das Prüfmuster Roggen-Gel bewirkte innerhalb des Prüfzeitraumes eine eindeutige Verbesserung des Hautzustandes unreiner Haut, wobei kein vollständiges Abklingen des Eindruckes der unreinen Haut beobachtet werden konnte. Die Probanden beschrieben eine positive Wirkung des Prüfmusters.

**Tab. 1:**

| Entzündliche Läsionen und Komedonen | | | | |
|---|---|---|---|---|
| entzündliche Läsionen | | | | |
| Proband | to | 2 Wochen | 4 Wochen | 6 Wochen |
| 1 | 0 | -8 | -2 | -15 |
| 2 | 0 | 0 | -4 | -3 |
| 3 | 0 | -5 | -6 | -2 |
| 4 | 0 | -7 | -7 | 0 |
| 5 | 0 | -3 | -6 | -7 |

| Komedonen | | | | |
|---|---|---|---|---|
| Proband | to | 2 Wochen | 4 Wochen | 6 Wochen |
| 1 | 0 | 1 | -1 | -3 |
| 2 | 0 | -6 | -3 | -5 |
| 3 | 0 | 0 | -1 | -1 |
| 4 | 0 | -7 | -1 | -3 |
| 5 | 0 | -2 | -2 | -3 |

**Tab. 2:**

| Randomisierung der Prüfmuster und Probandenspezifikation | | | | |
|---|---|---|---|---|
| Prob. | Geschlecht | Alter (y) | Areal (Gesichtshälfte) | |
| | | | links | rechts |
| 1 | w | 23,81 | behandelt | unbehandelt |
| 2 | m | 21,56 | unbehandelt | behandelt |
| 3 | m | 23,21 | behandelt | unbehandelt |
| 4 | w | 22,5 | unbehandelt | behandelt |
| 5 | m | 19,54 | behandelt | unbehandelt |

### Biometrie

### Zielgrößen

Die Zielgrößen der Hautpflegeprüfung sind die Hautfeuchtigkeit (Corneometrie) und die Hautmikrotopographie (Replika, R, DIN).

### Auswertung der Zielgrößen

Die Auswertung der Zielgrößen erfolgte durch Relativierung der Originaldaten der mit dem Prüfmuster behandelten Areale auf die unbehandelte Situation und den jeweiligen Ausgangswert (to). Es wurde der Mittelwert und die Standardabweichung der relativierten Daten bestimmt. Die Prüfung der Signifikanz von Unterschieden zwischen der unbehandelten und der behandelten Situation erfolgte mit der Software "STATISTICA". Zunächst wurde die Verteilung der Paardifferenzen der entsprechenden Paarvergleiche geprüft. Bei Normalverteilung der Paardifferenzen erfolgte die Signifikanzprüfung mittels zweiseitigem T-Test für abhängige Stichproben. Bei NichtNormalverteilung der Paardifferenzen wäre die Signifikanzprüfung mit dem Wilcoxon-Test für abhängige Stichproben erfolgt.

### Größe des Probandenkollektivs

Die Größe des Probandenkollektivs belief sich auf 15 ± 2.

### Diskussion

Es wurden die auf die unbehandelte Situation und den Ausgangszustand doppelt relativierten Daten ausgewertet, da diese die Veränderung der unbehandelten Kontrolle und die Unterschiede in der Ausgangssituation zwischen den unbehandelten und den behandelten Arealen berücksichtigen. Anhand der doppelt relativierten Daten zeichnet sich nach einer 7-tägigen Prüfmusterbehandlung eine leichte, aber signifikante Erhöhung der Hautfeuchtigkeit(um ca. 6 %) und eine leichte, aber signifikante Verringerung der Rauhigkeit (um ca. 7 %) gegenüber der unbehandelten Situation und der Ausgangssituation ab.

Signifikante Veränderungen hinsichtlich der Veränderung der Hautfeuchtigkeit und der Hautrauhigkeit konnten nach einer 21-tägigen Behandlung und der einwöchigen Behandlungspause nicht festgestellt werden. Das Prüfmuster Roggengel weist nur einen geringen befeuchtenden und glättenden Effekt auf die Haut auf.

Die klimatischen Verhältnisse im Prüfzeitraum spiegeln eine warme Sommerwetterlage mit Tagestemperaturen von ca. 16 °C bis 28 °C, viel Sonnenschein und einer relativ geringen Luftfeuchtigkeit wieder. Die erste Anwendungswoche war im Vergleich zu den anderen etwas kühler, bei geringerem Sonnenschein und einer etwas erhöhten Luftfeuchtigkeit. Die klimatischen Bedingungen an den Prüftagen waren unterschiedlich. Die Tagestemperaturen waren annähernd gleich, die Luftfeuchtigkeit schwankte um ca. 25 %. Die Meßklimabedingungen blieben während der 3 Prüftage konstant.

Aufgrund der Randomisierung der Prüfmuster starteten alle Prüfmuster mit den gleichen Ausgangsbedingungen.

Überprüfung der Einschlußkriterien Alle Probanden erfüllten die Einschlußkriterien.

### Meßergebnisse

### Hautfeuchtigkeit

Die Ausgangssituation stellt sich homogen dar, d.h. die unbehandelten und die zu behandelnden Areale weisen keine signifikanten Unterschiede hinsichtlich der Hautfeuchte auf.

Zum Zeitpunkt t₁ erhöhte sich durch die Prüfmusterbehandlung die Hautfeuchtigkeit im Mittel um ca. 6 % (± 12 %) gegenüber der unbehandelten Situation und dem Ausgangszustand. Dieser Unterschied ist signifikant. Nach der 3-wöchigen Behandlung ist im Mittel nur noch eine sehr leichte, aber nicht signifikante Erhöhung der Hautfeuchtemeßwerte durch die Prüfmusterbehandlung zu verzeichnen (ca. 2 %). Nach der einwöchigen Behandlungspause ist im Mittel die Hautfeuchte leicht, aber nicht signifikant erhöht gegenüber der unbehandelten Situation und dem Ausgangswert (ca. 5 %)

### Hautmikrotopographie

Die Originaldaten der Bestimmung der Hautrauhigkeit (Replika, R_{z} DIN) sind im Anhang unter, die auf die unbehandelte Situation und to relativierten Daten unter dargestellt.

Die Ausgangssituation ist nicht homogen, d.h. die unbehandelten Areale sind signifikant glatter als die zu behandelnden Hautareale.

Zum Zeitpunkt t₁ verringerte sich durch die Prüfmusterbehandlung die Rauhigkeit im Mittel um ca. 7,5 % (± 7 %) gegenüber der unbehandelten Situation und dem Ausgangszustand. Dieser Unterschied ist signifikant. Nach der 3-wöchigen Behandlung und der einwöchigen Behandlungspause ist im Mittel keine Verringerung der Rauheitsmeßwerte durch die Prüfmusterbehandlung mehr zu verzeichnen.

### Beispiel 3

Im folgenden wird die Analyse des erfindungsgemäßen Heil- und Pflegemittels angegeben.

Bei Voruntersuchungen zur Auftrennung einer Sauerteig-Wasser-Suspension mittels Zentrifugalsedimentation wurde ein 3-Phasen-System festgestellt.

Im Rahmen dieser Untersuchung sollte festgestellt werden, ob es sich bei der Mittelphase um eine stabile handelt, woraus sie aufgebaut sein könnte und ob es dort gegenüber des wäßrigen Überstandes zu einer Anreicherung wichtiger Inhaltsstoffe wie organische Säuren, Proteine und wasserlösliche Vitamine kommt. Auslegungshinweise für eine Separierung der Feststoffe sollten erlangt werden.

### Methoden

### Zentrifugation der Sauerteig-Suspension

### Verwendete Geräte:

Tischzentrifuge Hermie z 320
Laborzentrifuge: Hermie z 513
Rheometrische Untersuchungen der abgetrennten Phasen
Verwendetes Gerät: Rheometer: carri med csl (controi stress rheometer)
Methode: Fließkurvenermittlung mit vorgegebener Schubspannung und gemessener Schergeschwindigkeit bei einer Temperatur von 23 °C.

### Chemische Analysen

Probengewinnung: Um genügend Probe für die chemischen Analysen zu erhalten wurden 1600 g Sauerteigmischung bei 4300 Upm für 40 Minuten zentrifugiert. Die einzelnen Phasen wurden sorgfältig abpipettiert und für die externe Analyse eingefroren.

Alle chemischen Analysen erfolgten am Deutschen Institut für Lebensmitteltechnik. Methoden siehe Ergebnisse.

**Tab. 3:**

| Analysenergebnisse des klaren, wäßrigen Überstandes | | | |
|---|---|---|---|
| Parameter | Verfahren | Einheit | Meßwert |
| Rohprotein | Kjeldahl | g/1 00 g | 0,73 |
| Essigsäure | HPLC-Refr. | G/1 00 g | 0,06 |
| Milchsäure | HPLC-Refr. | G/1 00 g | 0,64 |
| Propionsäure | HPLC-Refr. | G/1 00 g | <0,01 |
| Buttersäure | HPLC-Refr. | G/1 00 g | <0,01 |
| Ascorbin/Dehydroascorbinsäure | HPLC- Fluoresz | mg/1 00 g | <5 |
| Vitamin B 2 | HPL-J-UVD | mg/1 00 g | < 0,5 |
| Vitamin B 6 (Pyridoxin) | HFL-C-UVD | mg/1 00 g | < 1 |
| Folsäure | HPLC-UVD | mg/100 g | < 2 |
| Ethanol | HPLC-Refr. | g/1 00.g | 0,07 |

**Tab. 4:**

| Analysenergebnisse des milchigen, gelartigen Überstandes (Mittelphase) | | | |
|---|---|---|---|
| Parameter | Verfahren | Einheit | Meßwert |
| Rohprotein | Kjeldahl | g/1 00 g | 0,83 |
| Essigsäure | HPLC-Refr. | g/1 00 g | 0,05 |
| Milchsäure | HPLC-Refr. | g/1 00 g | 0,64 |
| Propionsäure | HPLC-Refr. | g/1 00 g | < 0,01 |
| Buttersäure | HPLC-Refr.. | g/1 00 g | < 0,01 |
| Ascorbin-/Dehydroascorbinsäure | HPLC- Fluoresz. | Mg/100 g | < 5 |
| Vitamin B₂ | HPLC-UVD | mg/100 g | < 0,5 |
| Vitamin B₆ (Pyridoxin) | HPLC-UVD | mg/100 g | < 1 |
| Folsäure | HPLC-UVD | mg/100 g | < 2 |
| Ethanol | HPLC-Refr. | g/1 00 g | 0,07 |

### Beispiel 4

Das erfindungsgemäße Kosmetikmittel ist geeignet bei folgenden Erscheinungen eingesetzt zu werden.

### AKNE:

Fermentierter Rohstoff wurde in der Anfangsphase frisch (lebende Kulturen) auf die Gesichtshaut aufgetragen und nach abtrocknen der "Maske" mit warmem Wasser abgenommen.

Als Alternative kann auch eine pasteurisierte "Maske" verwendet werden.

Die Ergebnisse wurden von den Probanden, die beide Produkte angewendet haben, als nahezu identisch bewertet:

Rosige, gut durchblutete, weiche Haut, Aknebild war stark verbessert. In zwei Fällen wurde ein totales Abheilen beobachtet. Nach 3 Tagen setzte allerdings erneut die Bildung von Aknepusteln ein, die dann aber wieder mit einer erfindungsgemäßen Ferment-Regenerationsmaske behandelt werden konnten.

Bei Aknenarben konnten nach drei Behandlungen schon wesentliche Verbesserungen gestellt werden. Die Haut sah glatter aus und wirkte gepflegt und gut durchblutet (Behandlung siehe oben).

### KOPFHAUT:

Hier hat sich das erfindungsgemäße Kosmetikmittel in Form des Serums bei verschiedenen Problemen bewährt.

Eine typische Anwendung ist wie folgt:

Vor dem Waschen wird das Haar gescheitelt und das Produkt Strich für Strich einmassiert. Nach einer Einwirkzeit von 15 Minuten wäscht man mit einem milden Shampoo das Haar wie gewohnt.

Schuppen, entzündliche Partien, bis hin zu partiellem Haarausfall nach Ablösen größerer Kopfhautzonen konnten verbessert und um Teil sogar geheilt werden.

### Ergebnisse

### Abweichungen vom Prüfplan

Es traten keine Abweichungen vom Prüfplan auf.

### Unerwünschte Ereignisse 1 Ausfälle

Es traten keine unvorhergesehenen Ereignisse auf. 5 Probanden absolvierten die Prüfung korrekt und vollständig.

### Überprüfung der Einschlußkriterien

Alle Probanden erfüllten die Einschlußkriterien.

### Applikationszeiten der Prüfmuster

Die Applikationszeiten der Prüfmuster entsprachen den Anforderungen.

### Ergebnisse der visuellen Begutachtung

Es wurde die Verträglichkeit von 6 Prüfmustern, jeweils unverdünnt und in der Verdünnung 50 % anhand einer einmaligen Applikation der Prüfmuster untersucht. Dabei wurde ein Prüfmuster als auffällig bewertet, wenn das Ergebnis der visuellen Bewertung ≥ 1,0 lautete, d.h. mindestens eine leichte, punktförmige oder diffuse Rötung auftrat.

Die Prüfmuster Weizen-Serum, Weizen-Gel, Hafer-Gel (unverdünnt und 50 %) und die Prüfmuster Hafer-Serum und Roggen-Gel (unverdünnt) waren bei keinem Probanden auffällig.

Auf die Prüfmuster Hafer-Serum und Roggen-Gel in der Verdünnung 50 % und das Prüfmuster Roggen-Serum (unverdünnt und 50 %) reagierte jeweils 1 Proband (20 % aller Probanden) mit einer leichten punktförmigen oder diffusen Rötung. Das Maximum der Reaktion auf diese Prüfmuster trat 24 h nach Expositionsende auf. Die Reaktionen waren in allen Fällen auf das Applikationsareal begrenzt.

## Patentansprüche

1. Verfahren zur Herstellung eines Heil- und Pflegemittels für die Haut auf Basis eines ausgesäuerten Sauerteiges, wobei
1.1 einem Roggenmehl oder Roggenschrot ein natürlicher enzymatischer Starter wie α-Amylase und Wasser zugegeben wird, die Mischung einem Erhitzungsprozess auf 65 -75°C innerhalb von 3-5 Stunden ausgesetzt wird, wobei durch enzymatische Reaktionen eine starke Maltosebildung einsetzt,
1.2. eine weitere Zugabe von Roggenmehl oder Roggenschrot, Wasser sowie eines bakteriologischen Impfguts aus der Gruppe der heterofermentativen Milchsäurebakterien, vorzugsweise Lactobacillus DSM 6037 und Lactobacillus DSM 6129, erfolgt
1.3. und der Ansatz bis zur Einstellung der Stoffwechseltätigkeit der Mikroorganismen ausgesäuert wird.

2. Verfahren nach Anspruch 1, wobei dem Fermentationsprodukt ein zweites kohlehydrathaltiges Substrat und Wasser sowie ein bakteriologisches Impfgut aus der Gruppe der heterofermentativen Milchsäurebakterien zugegeben und die Mischung ausgesäuert wird,
- der letzte Verfahrensschritt ggf. ein oder mehrmals wiederholt wird, wobei die Mischung abschließend bis zur Einstellung jeglicher bakteriologischer Stoffwechselaktivität ausgesäuert wird und
- das Fermentationsprodukt einem stabilisierenden Erhitzungsprozess nahe der Siedetemperatur unterzogen wird.

3. Heil- und Pflegemittel für die Haut auf Basis eines ausgesäuerten Sauerteiges erhältlich nach dem Verfahren nach Anspruch 1 und/oder 2.

4. Heil- und Pflegemittel nach Anspruch 3, **dadurch gekennzeichnet, dass** das Heil- und Pflegemittel einen hohen Anteil auf fermentativem Wege gewonnenen Milchsäure von > 60 % (als Anteil der Gesamtsäuremenge) aufweist, wobei vor der Aussäuerung des Sauerteigs eine Behandlung mit α-Amylase erfolgt.

5. Heil- und Pflegemittel nach Anspruch 3 und/oder 4 mit in der Kosmetik üblichen Hilfs- und Trägerstoffen.

6. Heil- und Pflegemittel nach mindestens einem der Ansprüche 3 bis 5 in Form Pulvern, Roggenfluiden, Roggenseren, Roggengelen.

7. Kosmetikum enthaltend ein Heil- und Pflegemittel nach mindestens einem der Ansprüche 3 bis 6, in Form von Salben, Cremes, Lotionen, Gelen, Shampoos, Haarspülungen, Packungen, Lippenstiften, Gesichts- und Körpermasken, oder Kombinationen davon.

8. Verwendung von Heil- und Pflegemittel nach mindestens einem der Ansprüche 3 bis 6 in einem Kosmetikum nach Anspruch 7.
